(19) Europäisches Patentamt
European Patent Office
Office européen des brevets

(11) EP 2 921 162 B1

(12) # EUROPEAN PATENT SPECIFICATION

(45) Date of publication and mention of the grant of the patent:
**02.08.2017 Bulletin 2017/31**

(21) Application number: **13854887.0**

(22) Date of filing: **07.11.2013**

(51) Int Cl.:
***A61K 8/39*** *(2006.01)* ***A61K 8/06*** *(2006.01)*
***A61K 8/19*** *(2006.01)* ***A61Q 17/04*** *(2006.01)*

(86) International application number:
**PCT/JP2013/080066**

(87) International publication number:
**WO 2014/077174 (22.05.2014 Gazette 2014/21)**

(54) **OIL-IN-WATER EMULSION COMPOSITION**

ÖL-IN-WASSER-EMULSIONSVERBINDUNG

COMPOSITION DE TYPE ÉMULSION HUILE DANS EAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.11.2012 JP 2012250130**

(43) Date of publication of application:
**23.09.2015 Bulletin 2015/39**

(73) Proprietor: **Kao Corporation**
**Chuo-ku**
**Tokyo 103-8210 (JP)**

(72) Inventors:
- **MURATA, Takeshi**
**Kanagawa 2500002 (JP)**
- **YAMADA, Kenichi**
**Kanagawa 2500002 (JP)**

(74) Representative: **Hoffmann Eitle**
**Patent- und Rechtsanwälte PartmbB**
**Arabellastraße 30**
**81925 München (DE)**

(56) References cited:
**EP-A1- 1 604 648** **EP-A1- 2 497 457**
**EP-A2- 1 155 676** **FR-A1- 2 899 462**
**JP-A- H01 266 844** **JP-A- H05 163 116**
**JP-A- H07 291 827** **JP-A- H10 194 923**
**JP-A- 2006 008 796** **JP-A- 2012 001 527**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 921 162 B1

## Description

### Field of the Invention

The present invention relates to an oil-in-water emulsion composition stably containing an ultraviolet scattering agent.

### Background of the Invention

The importance of sunscreen measures in everyday life has been pointed out in recent years. Accordingly, oil-in-water emulsion-type sunscreen cosmetics which offer a refreshing feeling upon use and are suitable for continuous use have also been developed as cosmetics having a protective effect against ultraviolet rays.

In order to enhance the ultraviolet protective effect, an ultraviolet absorber and a metal oxide powder such as a zinc oxide or titanium dioxide powder are used in these oil-in-water emulsion cosmetics. Particularly, the metal oxide powder contained in a large amount not only causes powder aggregation, precipitation, and the like, over time but is disadvantageously responsible for reduction in stability over time resulting from reduced viscosity, separation of the emulsion, deposition, etc.

In order to solve such problems, an oil-in-water emulsion cosmetic has been proposed which has favorable powder dispersibility and stability over time and is excellent in a feeling upon use by use of a copolymer of acryloyldimethyl taurate and hydroxyethyl acrylate and a powder of titanium dioxide or the like (see Patent Literature 1). Also, an oil-in-water emulsion cosmetic has been proposed which has favorable stability over time and is excellent in a feeling upon use, transparency, and water resistance by use of zinc oxide, a crosslinked copolymer of acrylamide and 2-acrylamide-2-methylpropanesulfonic acid and/or an acrylic acid-sodium acryloyldimethyl taurate copolymer, a surfactant having an HLB of 10 or higher, a silicone oil, and water (see Patent Literature 2).

### Citation List

### Patent Literature

Patent Literature 1: JP-A-2006-8796
Patent Literature 2: JP-A-2005-272389
Patent Literature 3: WO 2011/136121
Patent Literature 4: WO 2011/155404

### Summary of the Invention

The present invention provides an oil-in-water emulsion composition comprising the following components (A) to (C):

(A) a polyoxyethylene alkyl or alkenyl ether which has an alkyl or alkenyl group having 20 or more and 24 or less carbon atoms and has an average molar number of ethylene oxide added of 1.5 or more and 4 or less,
(B) an oil agent which is liquid at 25°C, and
(C) a hydrophobized fine particle metal oxide powder.

The present invention also provides use of the oil-in-water emulsion composition as a sunscreen skin preparation for external use, and a method for producing the oil-in-water emulsion composition.

### Brief Description of the Drawings

[Figure 1] Figure 1 is an electron microscope photograph of a structure present on the emulsified particle interface (oil phase interface) of an oil-in-water emulsion composition of Example 1.
[Figure 2] Figure 2 is a microscope photograph of emulsified particles of Example 1.
[Figure 3] Figure 3 is a microscope photograph of emulsified particles of Comparative Example 1.
[Figure 4] Figure 4 is a microscope photograph of emulsified particles of Comparative Example 2.

[Figure 5] Figure 5 is a microscope photograph of emulsified particles of Comparative Example 3.
[Figure 6] Figure 6 is a microscope photograph of emulsified particles of Comparative Example 4.

Embodiments for Carrying out the Invention

The oil-in-water emulsion cosmetic described in Patent Literature 1 has poor powder dispersibility unless the powder is used in combination with a specific acrylic silicone as a dispersant in the oil-in-water emulsion cosmetic. Rather, the acrylic silicone causes a tight skin feeling, resulting in an inferior feeling upon use. Alternatively, the oil-in-water emulsion cosmetic described in Patent Literature 2 poorly spreads or stays during application and is thus unsatisfactory in terms of usability.

Thus, the present invention provides an oil-in-water emulsion composition which is excellent in the stability of a metal oxide powder contained and also in uniform applicability, is highly agreeable to the skin, spreads and stays well, is favorably usable with reduced feelings of stickiness and friction, exhibits excellent stability over time, and has a high moisturizing effect.

The present inventors found that a significant water evaporation inhibiting effect is obtained by use of a polyoxyethylene alkyl or alkenyl ether which has an alkyl or alkenyl group having 20 or more and 24 or less carbon atoms and has an average molar number of ethylene oxide added of 1.5 or more and 4 or less (hereinafter, this ether is referred to as polyoxyethylene alkyl ether (A)) for an aqueous composition in combination with a water-soluble polymer (Patent Literature 3). When the polyoxyethylene alkyl ether (A) is mixed into an oil phase containing a specific polar oil, the water evaporation inhibiting effect is disadvantageously reduced (Patent Literature 4). Accordingly, the present inventors conducted detailed study on the behavior of the polyoxyethylene alkyl ether (A) in the oil phase and consequently found that the polyoxyethylene alkyl ether (A) is present on the interface between the aqueous phase and the oil phase so as to form a structure. Also, a problem of an oil-in-water emulsion composition containing a hydrophobized fine particle metal oxide powder is that the hydrophobized fine particle metal oxide powder included in the oil phase tends to be heterogeneous due to its large emulsified particle size and is therefore less likely to maintain stability over time. The present inventors found that an oil-in-water emulsion composition which contains emulsified particles converted into fine particles, shows reduced aggregation and precipitation, has significantly improved stability, is further excellent in uniform applicability, is highly agreeable to the skin, spreads and stays well, offers reduced feelings of stickiness and friction, and exhibits an excellent moisturizing effect after application is obtained by allowing the oil phase to contain the polyoxyethylene alkyl ether (A). On the basis of these findings, the present invention was completed.

The oil-in-water emulsion composition of the present invention contains fine and uniform emulsified particles, is excellent in stability over time and also in uniform applicability, is highly agreeable to the skin, spreads and stays favorably, offers reduced feelings of stickiness and friction, and exhibits an excellent moisturizing effect (moisturizing effect brought about by the inhibition of water evaporation from the skin). The oil-in-water emulsion composition of the present invention can be preferably used for sunburn prevention.

Hereinafter, the constitution of the present invention will be described in detail.

The oil-in-water emulsion composition of the present invention (Example 1) was observed using a transmission electron microscope under conditions given below. As a result, a layer structure as shown in Figure 1 was confirmed on the surface of emulsified particles (oil phase interface) having a diameter of approximately 5 μm. Thus, the structure according to the present invention refers to such a layer structure locally present on the oil phase interface. Also, this layer structure was found by X-ray analysis to be a multilamellar vesicle formed by the polyoxyethylene alkyl ether (A).

[Measurement conditions]

Apparatus: transmission electron microscope (model JEM-1011 manufactured by JEOL Ltd.)
Condition: acceleration voltage of 100 kV
Sample preparation: freeze-fracture replica technique
Sample preparation apparatus: model JFD-9010 manufactured by JEOL Ltd.

The polyoxyethylene alkyl or alkenyl ether (A) used in the present invention has an alkyl or alkenyl group having 20 or more and 24 or less carbon atoms and an average molar number of ethylene oxide added of 1.5 or more and 4 or less.

The alkyl or alkenyl group in the Polyoxyethylene alkyl ether (A) may be linear or branched and is not limited by its structure. The alkyl or alkenyl group is preferably a linear or branched alkyl group, more preferably a linear alkyl group. The number of carbon atoms of the alkyl or alkenyl group is 20 or more and 24 or less, preferably 21 or more and 23 or less. A behenyl group having 22 carbon atoms is more preferred. When the number of carbon atoms of the alkyl or alkenyl group is less than 20, the resulting polyoxyethylene alkyl ether (A) fails to be present on the oil phase

interface so as to form a structure. The number of carbon atoms of the alkyl or alkenyl group exceeding 24 is not preferred for formulation because the resulting polyoxyethylene alkyl ether (A) is difficult to dissolve in the oil phase.

The average molar number of ethylene oxide added in the polyoxyethylene alkyl ether (A) is in the range of 1.5 or more and 4 or less, preferably 1.5 or more and 3 or less, more preferably 1.5 or more and 2.5 or less. An average molar number of ethylene oxide added of less than 1.5 is not preferred because the resulting polyoxyethylene alkyl ether (A) is highly crystalline and is thus difficult to dissolve in the oil phase. Alternatively, when the average molar number of ethylene oxide added exceeds 4, the resulting polyoxyethylene alkyl ether (A) is less likely to form a structure on the oil phase interface. Generally available polyoxyethylene alkyl ether (A) is a mixture having a very wide distribution centered on the desired degree of polymerization as to the molar number of ethylene oxide added. For the present invention, it is important that the average molar number of ethylene oxide added falls within the range mentioned above.

Examples of the polyoxyethylene alkyl ether (A) of the present invention include polyoxyethylene (2) arachyl ether, polyoxyethylene (3) arachyl ether, polyoxyethylene (4) arachyl ether, polyoxyethylene (2) behenyl ether, polyoxyethylene (3) behenyl ether, polyoxyethylene (4) behenyl ether, polyoxyethylene (2) carnaubyl ether, polyoxyethylene (3) carnaubyl ether, and polyoxyethylene (4) carnaubyl ether and preferably include polyoxyethylene (2) behenyl ether, polyoxyethylene (3) behenyl ether, and polyoxyethylene (4) behenyl ether. In this context, the polyoxyethylene alkyl ether (A) of the present invention may be used in combination with a polyoxyethylene alkyl ether other than those exemplified above as long as the average molar number of ethylene oxide added in the polyoxyethylene alkyl ether (A) used falls within the range.

The content of the polyoxyethylene alkyl ether (A) in the oil-in-water emulsion composition of the present invention with respect to the total amount of the composition is preferably 0.01% by mass or more, more preferably 0.05% by mass or more, even more preferably 0.1% by mass or more, and is preferably 15% by mass or less, more preferably 10% by mass or less, even more preferably 5% by mass or less. The specific range is preferably from 0.01 to 15% by mass, more preferably from 0.05 to 10% by mass, even more preferably from 0.1 to 5% by mass. This range is preferred because the resulting polyoxyethylene alkyl ether (A) tends to form a structure on the oil phase interface and can be easily contained in the oil phase.

The oil agent (B) which is liquid at 25°C used in the present invention is an oil agent in a state having fluidity at 1 atom at 25°C and includes an oil agent in a paste form. This oil agent encompasses a liquid organic ultraviolet absorber and an oil agent for use in ordinary cosmetic composition (oil agent for a cosmetic composition). The oil-in-water emulsion composition according to the present invention preferably contains a liquid organic ultraviolet absorber for enhancing the ultraviolet protective effect.

Examples of the liquid organic ultraviolet absorber include 2-ethylhexyl p-methoxycinnamate, 2-ethoxyethyl p-methoxycinnamate, isopropyl p-methoxycinnamatediisopropyl cinnamic acid ester mixtures, methyl bis(trimethylsiloxy)silylisopentyl trimethoxycinnamate, amyl p-dimethylaminobenzoate, 2-ethylhexyl p-dimethylaminobenzoate, ethylene glycol salicylate, 2-ethylhexyl salicylate, benzyl salicylate, homomenthyl salicylate, octocrylene, and dimethicodiethyl benzalmalonate.

Of them, 2-ethylhexyl p-methoxycinnamate, 2-ethylhexyl salicylate, homomenthyl salicylate, octocrylene, or dimethicodiethyl benzalmalonate is preferred. From the viewpoint of being able to improve the emulsified state and stability over time, the liquid organic ultraviolet absorber is more preferably 2-ethylhexyl p-methoxycinnamate, octocrylene, or dimethicodiethyl benzalmalonate, even more preferably 2-ethylhexyl p-methoxycinnamate.

An oil agent for a cosmetic composition other than the organic ultraviolet absorber contained in the liquid oil is not particularly limited as long as the oil agent for a cosmetic composition is liquid at 1 atom at 25°C. Specific examples thereof can include: hydrocarbon oils such as $\alpha$-olefin oligomer, liquid isoparaffin, liquid paraffin, isohexadecane, hydrogenated polyisobutene, and squalane; triglycerides such as glyceryl trioctanoate, avocado oil, olive oil, sesame oil, rice bran oil, safflower oil, soybean oil, corn oil, rapeseed oil, castor oil, cottonseed oil, and mink oil; fatty acids such as oleic acid and isostearic acid; ester oils such as isopropyl myristate, butyl myristate, isopropyl palmitate, ethyl oleate, ethyl linoleate, isopropyl linoleate, cetyl caprylate, hexyl laurate, decyl myristate, decyl oleate, oleyl oleate, isostearyl laurate, isotridecyl myristate, isocetyl myristate, isostearyl myristate, octyldodecyl myristate, octyl palmitate, isocetyl palmitate, isostearyl palmitate, propylene glycol dioleate, isodecyl oleate, isopropyl isostearate, cetyl 2-ethylhexanoate, stearyl 2-ethylhexanoate, propylene glycol dicaprate, propylene glycol dioleate, glyceryl tri-2-ethylhexanoate, glyceryl tri(caprylatecaprate), isononyl isononanoate, diisopropyl sebacate, and propylene glycol isostearate; branched or unsaturated higher alcohols such as 2-octyldodecanol, isostearyl alcohol, and oleyl alcohol; and silicone oils such as dimethylpolysiloxane, decamethyltetrasiloxane, dodecamethylpentasiloxane, and methylphenylpolysiloxane.

Of them, octyldodecyl myristate, isocetyl myristate, cetyl 2-ethylhexanoate, glyceryl tri-2-ethylhexanoate, glyceryl tri(caprylate-caprate), isononyl isononanoate, diisopropyl sebacate, propylene glycol isostearate, isohexadecane, squalane, hydrogenated polyisobutene, or dimethylpolysiloxane is preferred. From the viewpoint of being able to improve friction derived from the component (C), the oil agent for a cosmetic composition is more preferably diisopropyl sebacate, octyldodecyl myristate, or dimethylpolysiloxane, even more preferably dimethylpolysiloxane.

The content of the component (B) in the oil-in-water emulsion composition of the present invention with respect

to the total amount of the cosmetic composition is preferably 0.5% by mass or more, more preferably 1% by mass or more, even more preferably 3% by mass or more, and is preferably 30% by mass or less, more preferably 25% by mass or less, even more preferably 20% by mass or less, because a feeling of stickiness and friction can be reduced and the ultraviolet protective effect can be retained. Specifically, the content is preferably from 0.5 to 30% by mass, more preferably from 1 to 25% by mass, even more preferably from 3 to 20% by mass. Also, the content mass ratio of the liquid organic ultraviolet absorber in the liquid oil is preferably 0.5 or more, more preferably 0.6 or more, even preferably 0.7 or more, for reducing the liquid oil content while enhancing the ultraviolet protective effect.

The content mass ratio of the component (A) to the component (B), (A/B), is preferably 0.02 or more, more preferably 0.03 or more, and is preferably 1 or less, more preferably 0.5 or less, even more preferably 0.2 or less, in terms of feelings of stickiness and friction and stability over time. The specific range of A/B is preferably from 0.02 to 1, more preferably from 0.03 to 0.5, even more preferably from 0.03 to 0.2.

The metal oxide powder for use in the hydrophobized fine particle metal oxide powder (C) used in the present invention is not particularly limited as long as the metal oxide powder can be used in the field of pharmaceutical drugs, cosmetics, and foods. In terms of easy availability on the market, preferred examples thereof can include at least one selected from the group consisting of zinc oxide, titanium dioxide, cerium oxide, iron oxide, and chromium oxide. For sunburn prevention, one or two or more metal oxide powders selected from the group consisting of zinc oxide, titanium dioxide, and cerium oxide having a high ultraviolet scattering effect are preferably used. Also, these metal oxide powders can contain a +2-valent or higher trace element, and metals such as iron, zirconium, calcium, manganese, magnesium, and yttrium can be contained in the fine particle metal oxide powder singly or in an appropriate combination of two or more.

For example, FINEX-25, FINEX-50, and FINEX-75 (all manufactured by Sakai Chemical Industry Co., Ltd.), MZ500 series and MZ700 series (all manufactured by Tayca Corp.), and ZnO-350 (manufactured by Sumitomo Osaka Cement Co., Ltd.) are commercially available as the fine particle zinc oxide powder. For example, TTO-55 series and TTO-51 series (all manufactured by Ishihara Sangyo Kaisha, Ltd.), and JR series and JA series (manufactured by Tayca Corp.) are commercially available as the fine particle titanium dioxide powder. Alternatively, highly pure cerium sold by Nikki Corp. or AGC Seimi Chemical Co., Ltd. is included as the fine particle cerium oxide powder. Of them, a fine particle zinc oxide powder or a fine particle titanium dioxide powder is preferably used.

The average particle size of the fine particle metal oxide powder used in the present invention is preferably 0.01 μm or larger, more preferably 0.012 μm or larger, even more preferably 0.015 μm or larger, and is preferably 1 μm or smaller, more preferably 0.8 μm or smaller, even more preferably 0.5 μm or smaller. The specific average particle size is preferably in the range of from 0.01 to 1 μm, more preferably from 0.012 to 0.8 μm, even more preferably from 0.015 to 0.5 μm. The average particle size in this range is preferred because the resulting powder can be prevented from being aggregated, is stable, and secures the transparency of the preparation. In this context, the average particle size is measured by a laser diffraction/scattering method.

Examples of the shape of the fine particle metal oxide powder used in the present invention include spherical, rod-like, spindle-like, needle-like, and amorphous shapes. Any shape of the powder can be used as long as its average particle size falls within the range mentioned above.

The hydrophobization treatment of the fine particle metal oxide powder is not particularly limited and can be performed beforehand by various surface treatments, for example, fluorine compound treatment, silicone treatment, silicone resin treatment, pendant treatment, silane coupling agent treatment, titanium coupling agent treatment, oil agent treatment, N-acylated lysine treatment, polyacrylic acid treatment, metallic soap treatment, amino acid treatment, inorganic compound treatment, plasma treatment, mechanochemical treatment, and surface treatment with a silane compound or a silazane compound.

Preferred examples thereof include surface treatment using silicone or a silicone resin, treatment using methyl hydrogen polysiloxane or a methyl hydrogen polysiloxane-dimethylpolysiloxane copolymer represented by the formula (1) given below as a surface treatment agent, and treatment using a silane compound or a silazane compound as a surface treatment agent. Surface treatment using silicone or a silicone resin or surface treatment using methyl hydrogen polysiloxane is more preferred.

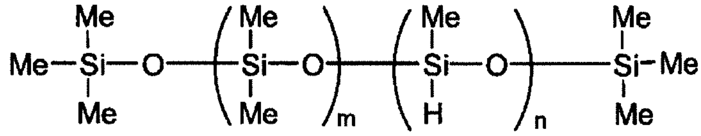 (1)

wherein m and n each represent an integer, and $1 \leq m+n \leq 60$.

Examples of the surface treatment using silicone or a silicone resin include a method as described in JP-B-3187440 which involves coating a metal oxide such as a zinc oxide powder with at least one silicone compound (except for a silane compound) selected from the group consisting of organopolysiloxanes and silicone resins in a non-gaseous

phase, followed by firing at a temperature of 600 to 950°C in an oxygen-containing atmosphere to coat the metal oxide surface with silicon oxide.

The silane compound or the silazane compound is preferably a silane compound or a silazane compound which has an alkyl or fluoroalkyl group having 1 to 20 carbon atoms and has reactivity with an inorganic oxide, and is specifically a silane compound represented by the general formula (2) given below or a silazane compound represented by the general formula (3) given below. These compounds can be used singly or in combination.

$$RR^1_nSiX_{3-n} \qquad (2)$$

wherein n represents an integer of 0 or 1; R represents an alkyl or fluoroalkyl group having 1 to 20 carbon atoms (which may be linear or branched); $R^1$ represents an alkyl group having 1 to 6 carbon atoms; and X represents a halogen atom or an alkoxy group.

$$R^2R^3R^4SiNHSiR^5R^6R^7 \qquad (3)$$

wherein $R^2$ to $R^7$, which are different from or the same with each other, each represent an alkyl or fluoroalkyl group having 1 to 20 carbon atoms (which may be linear or branched).

Specific examples of the silane compound include hexyltrimethoxysilane, octyltrimethoxysilane, decyltrimethoxysilane, octadecyltrimethoxysilane, octyltriethoxysilane, trifluoropropyltrimethoxysilane, and heptadecafluorodecyltrimethoxysilane. Of them, octyltriethoxysilane or octyltrimethoxysilane is preferred. Preferred examples of the silazane compound include hexamethyldisilazane. Features of the silane compound or the silazane compound are that the compound can facilitate uniform treatment, is readily supplied, and is inexpensive from an economic standpoint. When the fine particle metal oxide powder (C) surface-treated with the compound is contained in products, the resulting cosmetic compositions are preferred because of being excellent in properties such as dispersibility.

Examples of the method for the treatment with the silane compound or the silazane compound include a method which involves mixing the silane compound or the silazane compound with the metal oxide such as a zinc oxide powder in an organic solvent such as n-hexane, cyclohexane, or a lower alcohol, optionally pulverizing the mixture, then removing the organic solvent by heating or under reduced pressure, and subjecting the residue to heat treatment preferably at 80 to 250°C so that the silane compound or the silazane compound chemically reacts with the surface reactive group of the metal oxide such as zinc oxide.

Alternative examples thereof include a method as described in JP-A-2007-326902 which involves subjecting a cosmetic pigment to coating treatment with a specific polysiloxane compound, followed by surface treatment with alkylalkoxysilane in water.

The amount of the surface treatment agent in the coating on the fine particle metal oxide powder is preferably 3% by mass or more, more preferably 5% by mass or more, and is preferably 15% by mass or less, more preferably 10% by mass or less, with respect to the total amount of the powder used. The specific amount thereof in the coating is preferably from 3 to 15% by mass, more preferably from 5 to 10% by mass. The surface treatment agent of the amount in this range permits uniform coating of the surface of the powder and is prevented from being aggregated or deposited on the surface of the powder of zinc oxide or the like.

The content of the hydrophobized fine particle metal oxide powder (C) with respect to the total amount of the composition is preferably 0.1% by mass or more, more preferably 1% by mass or more, and is preferably 30% by mass or less, more preferably 25% by mass or less, even more preferably 20% by mass or less. The specific range is preferably from 0.1 to 30% by mass, more preferably from 1 to 25% by mass, even more preferably from 1 to 20% by mass. The powder having the content in this range has favorable dispersibility and is free from the problem of elevated viscosity of the preparation.

The component (C) preferably comprises 60% by mass or more of a hydrophobized fine particle zinc oxide powder as the hydrophobized fine particle metal oxide powder (C) because of leading to improvement in the UVA protective ability and improvement in stability at high temperatures.

In the oil-in-water emulsion composition of the present invention, the content mass ratio of the component (A) to the component (C), (A/C), is preferably 0.01 or more, more preferably 0.04 or more, more preferably 0.05 or more, even more preferably 0.1 or more, and is preferably 0.7 or less, more preferably 0.6 or less, more preferably 0.5 or less, even more preferably 0.4 or less, in terms of the conversion of the emulsified particles into fine particles and in terms of uniform applicability, spreadability and staying property, and a feeling of moisture. The specific range of A/C is preferably from 0.01 to 0.7, more preferably from 0.04 to 0.6, more preferably from 0.05 to 0.6, more preferably from 0.05 to 0.5, more preferably from 0.1 to 0.5, even more preferably from 0.1 to 0.4.

In the oil-in-water emulsion composition of the present invention, the content mass ratio of the component (B) to the component (C), (B/C), is preferably 0.62 or more, more preferably 0.65 or more, in terms of uniform applicability, a UV protective effect, spreadability and staying property, and a feeling of friction. Also, the content mass ratio (B/C) is

preferably 10 or less from the viewpoint of enhancing the UV protective effect. The specific range of B/C is preferably 0.62 or more, more preferably 0.65 or more, more preferably from 0.62 to 10, even more preferably from 0.65 to 10.

In the present invention, preferably, a water-soluble polymer (D) is further used for enhancing the dispersion stability of the oil phase. Examples of the water-soluble polymer used in the present invention include water-soluble cationic polymers, anionic polymers, nonionic polymers, and amphoteric polymers or bipolar polymers.

Specific examples of the cationic polymers include hydroxyethylcellulose having a O-[2-hydroxy-3-(trimethylammonio)propyl] chloride group (polyquaternium-10), (vinylpyrrolidone-dimethylaminomethylethyl methacrylate copolymer diethyl sulfate (polyquaternium-11), and methylvinylimidazolium chloride-vinylpyrrolidone copolymer.

Specific examples of the anionic polymers include carboxyvinyl polymer, carboxymethylcellulose, carrageenan, xanthan gum, polystyrene sulfonate, agar, ghatti gum, karaya gum, pectin, alginate salt, acrylic acid-alkyl methacrylate copolymer, (sodium acrylate/sodium acryloyldimethyl taurate) copolymer, acrylic acid or methacrylic acid derivatives such as alkali metal and ammonium salts of poly(acrylic acid) or acrylic acid or methacrylic acid, and hyaluronic acid or alkali metal salts thereof.

Specific examples of the nonionic polymers include cellulose ether such as hydroxybutylmethylcellulose, hydroxypropylcellulose, hydroxypropylmethylcellulose, ethylhydroxyethylcellulose, and hydroxyethylcellulose, propylene glycol alginate, polyacrylamide, poly(ethylene oxide), polyvinyl alcohol, polyvinyl pyrrolidone, hydroxypropyl guar gum, locust bean gum, amylose, hydroxyethyl amylose, starch and starch derivatives, and mixtures thereof.

Specific examples of the amphoteric polymers or bipolar polymers include octylacrylamide/acrylate/butylaminoethyl methacrylate copolymer, polyquaternium-47, and polyquaternium-43.

These water-soluble polymers can be used singly or in an appropriate combination of two or more thereof. In terms of easy applicability to various formulations, preferred examples thereof include one or two or more selected from the group consisting of carboxyvinyl polymer, acrylic acid-alkyl methacrylate copolymer, xanthan gum, hydroxypropylmethylcellulose, polyacrylamide, (sodium acrylate/sodium acryloyldimethyl taurate) copolymer, (hydroxyethyl acrylate/sodium acryloyldimethyl taurate) copolymer, (acrylamide/ammonium acrylate) copolymer, polyacrylate-13, and hyaluronic acid or an alkali metal salt thereof. One or two or more selected from the group consisting of carboxyvinyl polymer, acrylic acid-alkyl methacrylate copolymer, xanthan gum, hydroxypropylmethylcellulose, polyacrylamide, (sodium acrylate/sodium acryloyldimethyl taurate) copolymer, and hyaluronic acid or an alkali metal salt thereof are more preferred.

These components are commercially available.

Examples thereof include SEPIGEL 305 (polyacrylamide, hydrogenated polyisobutene (or (C13,14) isoparaffin), laureth-7, water), SEPINOV EMT 10 ((hydroxyethyl acrylate/sodium acryloyldimethyl taurate) copolymer), SIMULGEL NS ((hydroxyethyl acrylate/sodium acryloyldimethyl taurate) copolymer, squalane, polysorbate 60, water), SIMULGEL FL ((hydroxyethyl acrylate/sodium acryloyldimethyl taurate) copolymer, isohexadecane, polysorbate 60, water), SEPIPLUS S ((hydroxyethyl acrylate/sodium acryloyldimethyl taurate) copolymer, polyisobutene, PEG-7 trimethylolpropane coconut oil alkyl ether, water), SIMULGEL EG ((sodium acrylate/sodium acryloyldimethyl taurate) copolymer, isohexadecane, polysorbate 80, water), SEPIPLUS 265 ((acrylamide/ammonium acrylate) copolymer, polyisobutene, polysorbate 20, water), and SEPIPLUS 400 (polyacrylate-13, polyisobutene, polysorbate 20, water) sold by SEPPIC, and KELTROL (xanthan gum) sold by CP Kelco (the components contained are described within the parentheses).

The content of the component (D) with respect to the total amount of the composition is preferably 0.01% by mass or more, more preferably 0.05% by mass or more, and is preferably 5% by mass or less, more preferably 3% by mass or less. The specific range is preferably from 0.01 to 5% by mass, more preferably from 0.05 to 3% by mass. The content in the range is preferred because the stability of the preparation is maintained without a feeling of stickiness attributed to the water-soluble polymer.

In the present invention, preferably, the oil-in-water emulsion composition further comprises (E) a saturated monohydric alcohol having 1 to 3 carbon atoms, from the viewpoints of enhancing spreadability during application, reducing a feeling of stickiness, and enhancing water resistance and perspiration resistance. Specific examples of the component (E) include methyl alcohol, ethyl alcohol, propyl alcohol, and isopropyl alcohol.

The content of the component (E) is not particularly limited. The content of the component (E) with respect to the total amount of the composition is preferably 1% by mass or more, more preferably 3% by mass or more, even more preferably 5% by mass or more, and is preferably 25% by mass or less, more preferably 20% by mass or less, and even more preferably 15% by mass or less, from the viewpoint of enhancing spreadability during application, reducing a feeling of stickiness, and improving water resistance and perspiration resistance. The specific range is preferably from 1 to 25% by mass, more preferably from 3 to 20% by mass, even more preferably from 5 to 15% by mass.

In the present invention, preferably, the oil-in-water emulsion composition further comprises (F) a polyhydric alcohol in order to enhance moisturizing properties and spreadability during application. Examples of the component (F) include: glycols such as ethylene glycol, diethylene glycol, triethylene glycol, polyethylene glycol (average molecular weight: lower than 650), propylene glycol, dipropylene glycol, polypropylene glycol (average molecular weight: lower than 650), isoprene glycol, and 1,3-butylene glycol; glycerin, diglycerin, and polyglycerin. Of them, ethylene glycol,

diethylene glycol, propylene glycol, dipropylene glycol, or 1,3-butylene glycol can be preferably used. Dipropylene glycol is more preferred. These polyhydric alcohols can be used singly or in an appropriate combination of two or more thereof.

The content of the component (F) with respect to the total amount of the composition is preferably 0.1% by mass or more, more preferably 0.5% by mass or more, even more preferably 1% by mass or more, and is preferably 30% by mass or less, more preferably 20% by mass or less, more preferably 15% by mass or less, even more preferably 10% by mass or less, from the viewpoint of enhancing moisturizing properties and spreadability during application. The specific range is preferably from 0.1 to 30% by mass, more preferably from 0.5 to 20% by mass, more preferably from 0.5 to 15% by mass, more preferably from 1 to 15% by mass, even more preferably from 1 to 10% by mass.

In the oil-in-water emulsion composition of the present invention, the content of water with respect to the total amount of the composition is preferably 40% by mass or more, more preferably 45% by mass or more, and is preferably 80% by mass or less, more preferably 75% by mass or less, from the viewpoint of forming an oil-in-water emulsion composition excellent in stability over time. The specific range is preferably from 40 to 80% by mass, more preferably from 45 to 75% by mass.

In the oil-in-water emulsion composition of the present invention, various components usually contained in cosmetic compositions, for example, surfactants, lower alcohols, fluorine compounds, resins, thickeners, antibacterial or antiseptic agents, fragrances, moisturizers, salts, solvents, antioxidants, chelating agents, neutralizing agents, pH adjusters, insect repellents, and biologically active components, can be used in addition to the components mentioned above, within the range in which the effects of the present invention are not impaired.

The oil-in-water emulsion composition of the present invention is not particularly limited by its uses and can be preferably used in cosmetic compositions, pharmaceutical drugs, quasi-drugs, and the like. The emulsion composition of the present invention is preferably used as a skin preparation for external use of type which is retained on the skin without being washed off, because of its excellent moisture-occluding effect. The emulsion composition of the present invention is preferably used for sunburn prevention and is more preferably applied to sunscreens, suntan preparations, makeup base cosmetic compositions, foundations having ultraviolet protective ability, and the like.

The formulation of the oil-in-water emulsion composition of the present invention is applicable to, for example, liquid, emulsion, cream, paste, solid, and multilayer forms and is also applicable to sheet preparations, spray preparations, and mousse preparations.

The oil-in-water emulsion composition of the present invention can prevent the hydrophobized fine particle metal oxide powder from entering the aqueous phase, form fine emulsified particles, enhance the dispersibility of the oil phase, and produce excellent stability over time by containing the components (A) to (C) in the oil phase. As for the structure of the emulsified particles in the oil-in-water emulsion composition of the present invention, it appears that the component (A) is present on the emulsified particle (oil phase) interface, as described above, so as to form a soft structure (multilamellar vesicle structure), and the component (C) is stably dispersed in the component (B) in the oil phase. Such a structure formed converts the emulsified particles into fine particles, reduces aggregation and precipitation, significantly improves stability, and secures excellent uniform applicability, high agreeability to the skin, favorable spreadability and staying property, reduced feelings of stickiness and friction, and a favorable moisturizing effect after application.

The average emulsified particle size of the oil-in-water emulsion composition of the present invention is preferably 1 $\mu$m or larger, and is preferably 30 $\mu$m or smaller, more preferably 25 $\mu$m or smaller, even more preferably 20 $\mu$m or smaller, in terms of stability over time and uniform application. The specific range is preferably from 1 to 30 $\mu$m, more preferably from 1 to 25 $\mu$m, even more preferably from 1 to 20 $\mu$m. In this context, the average particle size of the emulsified particles can be determined from a number-based mean by the repetitive measurement of the particle sizes of 20 arbitrary particles in any field of view in a microscope photograph.

In order to enhance the orientation of the polyoxyethylene alkyl ether (A) toward the oil phase interface, the oil-in-water emulsion composition of the present invention is preferably produced by dissolving an oil-phase component containing the components (A) to (C) by heating at a temperature exceeding 45°C and emulsifying the oil-phase component with an aqueous-phase component of 15 to 40°C. More specifically, the components (A) to (C) are dissolved, together with other oil-phase components, by heating at a temperature exceeding 45°C, preferably a temperature of 50 to 80°C, and the mixture is uniformly stirred and mixed. Then, the oil-phase component is uniformly mixed with an aqueous-phase component of 15 to 40°C, preferably 20 to 30°C, by a method such as phase-inversion emulsification or non-phase-inversion emulsification to form an oil-in-water emulsion composition. This emulsification is preferably performed by the gradual addition of the oil-phase component into the aqueous-phase component. More preferably, the uniformly stirred oil phase is gradually added into the aqueous phase (including the component (D)), and the mixture is uniformly stirred and mixed, and then cooled to room temperature to produce an emulsion composition. The polyoxyethylene alkyl ether (A) forms a structure on the oil phase interface at the same time with the addition of the oil phase into the aqueous phase. As a result, a stable oil-in-water emulsion composition can be obtained.

In relation to the embodiments mentioned above, the present invention further discloses the following embodiments:

<1> An oil-in-water emulsion composition comprising the following components (A) to (C):

(A) a polyoxyethylene alkyl or alkenyl ether which has an alkyl or alkenyl group having 20 or more and 24 or less carbon atoms and has an average molar number of ethylene oxide added of 1.5 or more and 4 or less,
(B) a liquid oil, and
(C) a hydrophobized fine particle metal oxide powder.

<2> The oil-in-water emulsion composition according to <1>, wherein the number of carbon atoms of the alkyl or alkenyl group in the component (A) is preferably 21 or more and 23 or less, more preferably 22.
<3> The oil-in-water emulsion composition according to <1> or <2>, wherein the average molar number of ethylene oxide added in the component (A) is preferably 1.5 or more and 3 or less, more preferably 1.5 or more and 2.5 or less.
<4> The oil-in-water emulsion composition according to any of <1> to <3>, wherein the component (A) is preferably at least one selected from the group consisting of polyoxyethylene (2) arachyl ether, polyoxyethylene (3) arachyl ether, polyoxyethylene (4) arachyl ether, polyoxyethylene (2) behenyl ether, polyoxyethylene (3) behenyl ether, polyoxyethylene (4) behenyl ether, polyoxyethylene (2) carnaubyl ether, polyoxyethylene (3) carnaubyl ether, and polyoxyethylene (4) carnaubyl ether, more preferably at least one selected from the group consisting of polyoxyethylene (2) behenyl ether, polyoxyethylene (3) behenyl ether, and polyoxyethylene (4) behenyl ether.
<5> The oil-in-water emulsion composition according to any of <1> to <4>, wherein the content of the component (A) with respect to the total amount of the composition is preferably 0.01% by mass or more, more preferably 0.05% by mass or more, even more preferably 0.1% by mass or more, and is preferably 15% by mass or less, more preferably 10% by mass or less, even more preferably 5% by mass or less.
<6> The oil-in-water emulsion composition according to any of <1> to <5>, wherein the content of the component (A) with respect to the total amount of the composition is preferably from 0.01 to 15% by mass, more preferably from 0.05 to 10% by mass, even more preferably from 0.1 to 5% by mass.
<7> The oil-in-water emulsion composition according to any of <1> to <6>, wherein the component (B) preferably comprises a liquid organic ultraviolet absorber and an oil agent for a cosmetic composition.
<8> The oil-in-water emulsion composition according to <7>, wherein the liquid organic ultraviolet absorber is preferably at least one selected from the group consisting of 2-ethylhexyl p-methoxycinnamate, 2-ethoxyethyl p-methoxycinnamate, an isopropyl p-methoxycinnamate-diisopropyl cinnamic acid ester mixture, methyl bis(trimethylsiloxy)silylisopentyl trimethoxycinnamate, amyl p-dimethylaminobenzoate, 2-ethylhexyl p-dimethylaminobenzoate, ethylene glycol salicylate, 2-ethylhexyl salicylate, benzyl salicylate, homomenthyl salicylate, octocrylene, and dimethicodiethyl benzalmalonate.
<9> The oil-in-water emulsion composition according to <7> or <8>, wherein the oil agent for a cosmetic composition is preferably at least one selected from the group consisting of a hydrocarbon oil, a triglyceride, a fatty acid, an ester oil, a higher alcohol, and a silicone oil.
<10> The oil-in-water emulsion composition according to any of <1> to <9>, wherein the content of the component (B) with respect to the total amount of the composition is preferably 0.5% by mass or more, more preferably 1% by mass or more, even more preferably 3% by mass or more, and is preferably 30% by mass or less, more preferably 25% by mass or less, even more preferably 20% by mass or less.
<11> The oil-in-water emulsion composition according to any of <1> to <10>, wherein the content of the component (B) with respect to the total amount of the composition is preferably from 0.5 to 30% by mass, more preferably from 1 to 25% by mass, even more preferably from 3 to 20% by mass.
<12> The oil-in-water emulsion composition according to any of <7> to <11>, wherein the content mass ratio of the liquid organic ultraviolet absorber in the component (B) is preferably 0.5 or more, more preferably 0.6 or more, even more preferably 0.7 or more.
<13> The oil-in-water emulsion composition according to any of <1> to <12>, wherein the content mass ratio of the component (A) to the component (B), (A/B), is preferably 0.02 or more, more preferably 0.03 or more, and is preferably 1 or less, more preferably 0.5 or less, even more preferably 0.2 or less.
<14> The oil-in-water emulsion composition according to any of <1> to <13>, wherein the content mass ratio of the component (A) to the component (B), (A/B), is preferably from 0.02 to 1, more preferably from 0.03 to 0.5, even more preferably from 0.03 to 0.2.
<15> The oil-in-water emulsion composition according to any of <1> to <14>, wherein the component (C) is preferably a hydrophobization product of one or two or more metal oxide powders selected from the group consisting of zinc oxide, titanium dioxide, and cerium oxide.
<16> The oil-in-water emulsion composition according to any of <1> to <15>, wherein the average particle size of the component (C) is preferably 0.01 μm or larger, more preferably 0.012 μm or larger, even more preferably 0.015 μm or larger, and is preferably 1 μm or smaller, more preferably 0.2 μm or smaller, even more preferably 0.1 μm or smaller.

<17> The oil-in-water emulsion composition according to any of <1> to <16>, wherein the average particle size of the component (C) is preferably from 0.01 to 1 μm, more preferably from 0.012 to 0.2 μm, even more preferably from 0.015 to 0.1 μm.

<18> The oil-in-water emulsion composition according to any of <1> to <17>, wherein a hydrophobization treatment of the fine particle metal oxide powder is preferably fluorine compound treatment, silicone treatment, silicone resin treatment, pendant treatment, silane coupling agent treatment, titanium coupling agent treatment, oil agent treatment, N-acylated lysine treatment, polyacrylic acid treatment, metallic soap treatment, amino acid treatment, inorganic compound treatment, plasma treatment, mechanochemical treatment, or surface treatment with a silane compound or a silazane compound, more preferably at least one surface treatment selected from a surface treatment using silicone or a silicone resin and a surface treatment using methyl hydrogen polysiloxane.

<19> The oil-in-water emulsion composition according to any of <1> to <18>, wherein the amount of the hydrophobization of the component (C) with respect to the total amount of the powder is preferably 3% by mass or more, more preferably 5% by mass or more, and is preferably 15% by mass or less, more preferably 10% by mass or less.

<20> The oil-in-water emulsion composition according to any of <1> to <19>, wherein the content of the component (C) with respect to the total amount of the composition is preferably 0.1% by mass or more, more preferably 1% by mass or more, and is preferably 30% by mass or less, more preferably 25% by mass or less, even more preferably 20% by mass or less.

<21> The oil-in-water emulsion composition according to any of <1> to <20>, wherein the content of the component (C) with respect to the total amount of the composition is preferably from 0.1 to 30% by mass, more preferably from 1 to 25% by mass, even more preferably from 1 to 20% by mass.

<22> The oil-in-water emulsion composition according to any of <1> to <21>, wherein the component (C) preferably comprises 60% by mass or more of a hydrophobized fine particle zinc oxide powder.

<23> The oil-in-water emulsion composition according to any of <1> to <22>, wherein the content mass ratio of the component (A) to the component (C), (A/C), is preferably 0.01 or more, more preferably 0.04 or more, even more preferably 0.1 or more, and is preferably 0.7 or less, more preferably 0.6 or less, even more preferably 0.4 or less.

<24> The oil-in-water emulsion composition according to any of <1> to <23>, wherein the content mass ratio of the component (A) to the component (C), (A/C), is preferably from 0.01 to 0.7, more preferably from 0.04 to 0.6, even more preferably from 0.1 to 0.4.

<25> The oil-in-water emulsion composition according to any of <1> to <24>, further comprising (D) a water-soluble polymer.

<26> The oil-in-water emulsion composition according to <25>, wherein the component (D) is preferably one or two or more selected from the group consisting of carboxyvinyl polymer, acrylic acid-alkyl methacrylate copolymer, xanthan gum, hydroxypropylmethylcellulose, polyacrylamide, (sodium acrylate/sodium acryloyldimethyl taurate) copolymer, (hydroxyethyl acrylate/sodium acryloyldimethyl taurate) copolymer, (acrylamide/ammonium acrylate) copolymer, polyacrylate-13, and hyaluronic acid or an alkali metal salt thereof, more preferably one or two or more selected from the group consisting of carboxyvinyl polymer, acrylic acid-alkyl methacrylate copolymer, xanthan gum, hydroxypropylmethylcellulose, polyacrylamide, (sodium acrylate/sodium acryloyldimethyl taurate) copolymer, and hyaluronic acid or an alkali metal salt thereof.

<27> The oil-in-water emulsion composition according to <25> or <26>, wherein the content of the component (D) with respect to the total amount of the composition is preferably 0.01% by mass or more, more preferably 0.05% by mass or more, and is preferably 5% by mass or less, more preferably 3% by mass or less.

<28> The oil-in-water emulsion composition according to any of <25> to <27>, wherein the content of the component (D) with respect to the total amount of the composition is preferably from 0.01 to 5% by mass, more preferably from 0.05 to 3% by mass.

<29> The oil-in-water emulsion composition according to any of <1> to <28>, wherein the content of water with respect to the total amount of the composition is preferably 40% by mass or more, more preferably 45% by mass or more, and is preferably 80% by mass or less, more preferably 75% by mass or less.

<30> The oil-in-water emulsion composition according to any of <1> to <29>, wherein the composition is preferably used for sunburn prevention.

<31> The oil-in-water emulsion composition according to any of <1> to <30>, wherein the average emulsified particle size of the oil-in-water emulsion composition is preferably 1 μm or larger, and is preferably 30 μm or smaller, more preferably 25 μm or smaller, even more preferably 20 μm or smaller.

<32> The oil-in-water emulsion composition according to any of <1> to <31>, wherein the average particle size of the oil-in-water emulsion composition is preferably from 1 to 30 μm, more preferably from 1 to 25 μm, even more preferably from 1 to 20 μm.

<33> The oil-in-water emulsion composition according to any of <1> to <32>, wherein the composition is obtained by dissolving an oil-phase component comprising the components (A) to (C) by heating at a temperature preferably exceeding 45°C, more preferably of 50 to 80°C and emulsifying the oil-phase component with an aqueous-phase

component of preferably 15 to 40°C, more preferably 20 to 30°C.

<34> The oil-in-water emulsion composition according to <33>, wherein the emulsification is preferably performed by the gradual addition of the oil-phase component into the aqueous-phase component.

<35> Use of an oil-in-water emulsion composition according to any of <1> to <34> as a sunscreen skin preparation for external use.

<36> A method for producing an oil-in-water emulsion composition, comprising dissolving an oil-phase component containing the following components (A) to (C) by heating at a temperature exceeding 45°C and emulsifying the oil-phase component with an aqueous-phase component having a temperature of 15 to 40°C:

(A) a polyoxyethylene alkyl or alkenyl ether which has an alkyl or alkenyl group having 20 or more and 24 or less carbon atoms and has an average molar number of ethylene oxide added of 1.5 or more and 4 or less,
(B) a liquid oil, and
(C) a hydrophobized fine particle metal oxide powder.

<37> The method for producing an oil-in-water emulsion composition according to <36>, wherein the emulsification is performed by the gradual addition of the oil-phase component into the aqueous-phase component.

Examples

Hereinafter, the present invention will be described in more detail with reference to Examples. However, the present invention is not intended to be limited by them.

Methods for water resistance test and sensory evaluation used in Examples and Comparative Examples below will be described for illustrative purposes.

(1) Evaluation of emulsified state

Each sample shown in Table 1 was observed under a microscope immediately after production and evaluated according to criteria given below. When substantially spherical homogeneous emulsified particles were formed during the microscopic observation, the average particle size of these emulsified particles were determined from a number-based mean by the repetitive measurement of the particle sizes of 20 arbitrary particles in any field of view in a microscope photograph.

[Criteria for determining emulsified state by microscopic observation]

3: The powder entered the inner phase (oil phase), and substantially spherical homogeneous emulsified particles were formed.
2: The shapes and sizes of the emulsified particles were not uniform, though the powder entered the internal phase.
1: The powder leaked to the outer phase (aqueous phase).

(2) Test on occluding properties

A hydrophilic membrane filter (MF-Millipore GSWP02500, manufactured by Millipore Japan Co., Ltd.; pore diameter: 0.22 μm, the filter was cut into a diameter of 15 mm and then used) was added to 100 μl of each sample shown in Table 1 or 2. The filter was thoroughly dried on a hot plate of 37°C to prepare a dried membrane filter of the sample. 5 g of pure water was placed in a cell, which was then covered with the dried membrane filter of the sample. Change in weight was measured at a humidity of 30% and a temperature of 30°C. The results were plotted as absolute values when n in (nX + m wherein X represents a time (h)) calculated by the least-squares method was defined as a water evaporation rate (unit: mg/h). A mean of three measurements per sample was determined. In this context, the water evaporation rate means that evaporation is more suppressed at a smaller water evaporation rate.

(3) Uniform application evaluation

Each sample shown in Table 1 or 2 was uniformly applied at a proportion of 2 mg/cm$^2$ on a PMMA sheet and then dried. After the drying of the sample, the transmittances (%) of absorption spectra (wavelength: 370 nm) in 8 predetermined areas of the sample on the PMMA sheet were measured using an SPF analyzer (SPF 290S plus, manufactured by Optometricus USA, Inc.). The difference between the maximum and minimum values (maximum value -

minimum value) of the transmittances of the 8 areas was calculated. A smaller value of the difference was evaluated as more highly uniform applicablity.

(4) Test on UV protective ability

Each sample and a sample of Reference Example 1 was separately applied at a proportion of 2 mg/cm$^2$ onto a PMMA sheet and then dried. Then, the ultraviolet protective ability was measured using an SPF analyzer (SPF 290S plus, manufactured by Optometricus USA, Inc.) in 8 predetermined areas of the sample on the PMMA sheet. The rate of improvement in the UV protective ability of each sample compared with the sample of Reference Example was determined according to the following expression, and evaluation was conducted according to evaluation criteria given below:

$$\text{Rate of improvement in UV protective ability (\%)} = (\text{Average SPF value of the sample}) / (\text{Average SPF value of the sample of Reference Example 1}) \times 100.$$

[Criteria for evaluating rate of improvement in UV protective ability]

4: Exceeding 110%
3: 90% or more and less than 110%
2: 70% or more and less than 90%
1: Less than 70%

(5) Sensory evaluation

10 expert panelists actually used each sample shown in Table 2 and evaluated its usage characteristics [spreadability and staying property (uniform spreading without unevenness) and the presence or absence of a feeling of moisture, a feeling of stickiness, and a feeling of friction] according to evaluation criteria given below. Average scores are shown in the table.

(Evaluation criteria)

(a) Spreadability and staying property

5: Very good spreadability and staying property
4: Good spreadability and staying property
3: Fair spreadability and staying property
2: Poor spreadability and staying property
1: Very poor spreadability and staying property

(b) Feeling of moisture

5: Very strong moist feeling of the skin
4: Strong moist feeling of the skin
3: Fair
2: Weak moist feeling of the skin
1: Very weak moist feeling of the skin

(c) Feeling of stickiness

5: Very weak feeling of stickiness
4: Weak feeling of stickiness
3: Fair
2: Strong feeling of stickiness
1: Very strong feeling of stickiness

(d) Feeling of friction

5: Very weak feeling of friction
4: Weak feeling of friction
3: Fair
2: Strong feeling of friction
1: Very strong feeling of friction

Hereinafter, each hydrophobized fine particle metal oxide powder used in the present invention will be described.

(Production Example 1: Production of octyltriethoxysilane-surface treaded flaky powder of zinc oxide)

A slurry consisting of 93 parts by mass of a flaky powder of zinc oxide (average particle size: 0.3 μm, average particle thickness: 0.032 μm, plate ratio: 9, iron element content: 0.01 mol%), 7 parts by mass of octyltriethoxysilane, and toluene was prepared and then crushed and pulverized using a bead mill (DYNO-MILL manufactured by Shinmaru Enterprises Corp.).
Subsequently, toluene was distilled off by heating under reduced pressure. Then, the residue was heat-treated at 150°C for 4 hours using a blast air-type drier to obtain an octyltriethoxysilane-treated flaky powder of zinc oxide.

(Production Example 2: Production of methyl hydrogen polysiloxane-surface treated fine particle zinc oxide powder)

A slurry consisting of 95 parts by mass of a fine particle zinc oxide powder (substantially spherical, average particle size: 20 nm), 5 parts by mass of methyl hydrogen polysiloxane (KF-99P, manufactured by Shin-Etsu Chemical Co., Ltd.), and isopropyl alcohol was prepared and then well stirred and crushed. Then, the solvent was distilled off by heating under reduced pressure. The residue was heat-treated at 150°C for 4 hours in air to obtain a methyl hydrogen polysiloxane-surface treated fine particle zinc oxide powder.

(Production Example 3: Production of octyltriethoxysilane-surface treated fine particle titanium dioxide powder)

A slurry consisting of 93 parts by mass of a fine particle titanium dioxide powder (substantially spherical, average particle size: 0.017 μm), 7 parts by mass of octyltriethoxysilane, and toluene was prepared and then crushed and pulverized using a bead mill (DYNO-MILL manufactured by Shinmaru Enterprises Corp.). Subsequently, toluene was distilled off by heating under reduced pressure. Then, the residue was heat-treated at 150°C for 4 hours using a blast air-type drier to obtain an octyltriethoxysilane-surface treated titanium oxide powder.

(Production Example 4: Production of methyl hydrogen polysiloxane-surface treated fine particle titanium dioxide powder)

A slurry consisting of 95 parts by mass of a fine particle titanium dioxide powder (substantially spherical, average particle size: 0.017 μm), 5 parts by mass of methyl hydrogen polysiloxane (KF-99P, manufactured by Shin-Etsu Chemical Co., Ltd.), and isopropyl alcohol was prepared and then well stirred and crushed. Then, the solvent was distilled off by heating under reduced pressure. The residue was heat-treated at 160°C for 4 hours in air to obtain a methyl hydrogen polysiloxane-surface treated fine particle titanium dioxide powder.

[Study on type of polyoxyethylene alkyl ether]

Example 1 and Comparative Examples 1 to 4

Each oil-in-water sunscreen was prepared according to the formulation shown in Table 1. The tests described above were carried out using these samples. The test results are also shown in Table 1.

[Table 1]

| Component | | | Example 1 | Comparative Example 1 | Comparative Example 2 | Comparative Example 3 | Comparative Example 4 |
|---|---|---|---|---|---|---|---|
| 1 | A | Polyoxyethylene (2) behenyl ether | 1 | | | | |
| 2 | | Polyoxyethylene (5) behenyl ether | | | 1 | | |
| 3 | | Polyoxyethylene (2) stearyl ether | | | | 1 | |
| 4 | | Polyoxyethylene (2) cetyl ether | | | | | 1 |
| 5 | B | Dimethicone (kinetic viscosity (25°C) 6 $mm^2/s$) | 5.5 | 5.5 | 5.5 | 5.5 | 5.5 |
| 6 | B | 2-Ethylhexyl p-methoxycinnamate *1 | 7.5 | 7.5 | 7.5 | 7.5 | 7.5 |
| 7 | B | Octocrylene *2 | 0.2 | 0.2 | 0.2 | 0.2 | 0.2 |
| 8 | C | Octyltriethoxysilane-surface treated flaky powder of zinc oxide (Production Example 1) | 4 | 4 | 4 | 4 | 4 |
| 9 | C | Methyl hydrogen polysiloxane-surface treated fine particle zinc oxide powder (Production Example 2) | 6 | 6 | 6 | 6 | 6 |
| 10 | | Glycerin | 2 | 2 | 2 | 2 | 2 |
| 11 | | Ethanol | 5 | 5 | 5 | 5 | 5 |
| 12 | D | (Sodium acrylate/sodium acryloyldimethyl taurate) copolymer *3 | 1.5 | 1.5 | 1.5 | 1.5 | 1.5 |
| 13 | D | Xanthan gum | 0.1 | 0.1 | 0.1 | 0.1 | 0.1 |
| 14 | | Disodium edetate | 0.02 | 0.02 | 0.02 | 0.02 | 0.02 |
| 15 | | Pure water | Balance | Balance | Balance | Balance | Balance |
| | | | | | | | |
| Evaluation | | Evaluation of emulsified state | A (7.4 µm) | A (47.5 µm) | A (22.9 µm) | B | B |
| | | Evaluation of occluding properties | 4.3 | 5.2 | 5.3 | 5.4 | 5.5 |
| | | Uniform application evaluation | 3.2 | 11.5 | 9.3 | 9.4 | 10.1 |

*1 Uvinul MC-80 (manufactured by BASF Japan Ltd.)
*2 PARSOL 340 (manufactured by DSM Nutrition Japan K.K.)
*3 SIMULGEL EG (manufactured by SEPPIC)

(Production method)

a: The components (1) to (9) are dissolved by heating at 70°C and uniformly mixed.
b: The components (10) to (15) are uniformly mixed at room temperature (25°C ± 3°C).
c: While the product obtained in the step b is stirred, the product obtained in the step a is gradually added thereto. The mixture is uniformly mixed.
d: The mixture obtained in the step c is cooled to room temperature.

In the sample of Example 1 using polyoxyethylene (2) behenyl ether, the powder entered the inner phase (oil phase), and homogeneous and fine emulsified particles (average emulsified particle size: 7.4 μm) were obtained (Figure 2). This sample was also excellent in uniform applicability. In addition, its moisture-occluding effect after application was also excellent. By contrast, the sample of Comparative Example 1 free from polyoxyethylene (2) behenyl ether had larger emulsified particles than those of the sample of Example 1 (average emulsified particle size: 47.5 μm) (Figure 3) and had poor uniform applicability, though the powder entered the inner phase (oil phase) to form relatively homogeneous emulsified particles.

The samples of Comparative Examples 2 to 4 in which polyoxyethylene (2) behenyl ether was replaced with polyoxyethylene (5) behenyl ether, polyoxyethylene (2) stearyl ether, and polyoxyethylene (2) cetyl ether, respectively, had a large emulsified particle size than that of the sample of Example 1 or nonuniform shapes and sizes of emulsified particles, resulting in poor uniform applicability (Figures 4 to 6). Their moisture-occluding effects after application were almost equal to the effect obtained in Comparative Example 1. The addition of polyoxyethylene (5) behenyl ether, polyoxyethylene (2) stearyl ether, or polyoxyethylene (2) cetyl ether did not bring out the moisture-occluding effect.

[Study on content ratio]

Examples 2 to 16 and Reference Example 1

[Table 2]

| | | Component | Example 2 | Example 3 | Example 4 | Example 5 | Example 6 | Example 7 | Example 8 | Example 9 | Example 10 | Example 11 | Example 12 | Example 13 | Example 14 | Example 15 | Example 16 | Reference Example 1 |
|---|---|---|---|---|---|---|---|---|---|---|---|---|---|---|---|---|---|---|
| 1 | A | Polyoxyethylene (2) behenyl ether | 1 | 1 | 1 | 1 | 1 | 5 | 1 | 1 | 1 | 1 | 0.1 | 0.5 | 8 | 0.5 | 8 | 1 |
| 2 | B | Dimethicone (kinetic viscosity (25°C) 6 $mm^2/s$) | 5.5 | 5.5 | 5.5 | 5.5 | 5.5 | 5.5 | 4.5 | 15 | 5.5 | 5.5 | 5.5 | 5.5 | 5.5 | 5.5 | 4 | 5.5 |
| 3 | B | 2-Ethylhexyl p-methoxycinnamate *1 | 7.5 | 7.5 | 7.5 | 7.5 | 7.5 | 7.5 | 1.5 | 7.5 | 7.5 | 7.5 | 7.5 | 7.5 | 7.5 | 7.5 | 3.5 | 7.5 |
| 4 | B | Octocrylene *2 | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 |
| 5 | C | Octyltriethoxysilane-surface treated flaky powder of zinc oxide (Production Example 1) | 12 | 9 | 2.4 | 2 | 1.6 | 4 | 4 | 4 | | 12 | 12 | 12 | 4 | | 6 | 4 |
| 6 | C | Methyl hydrogen polysiloxane-surface treated fine particle zinc oxide powder (Production Example 2) | 8 | 6 | 3.6 | 3 | 2.4 | 6 | 6 | 6 | | | 8 | 8 | 6 | 1 | 5 | 6 |
| 7 | C | Octyltriethoxysilane-surface treated fine particle titanium dioxide powder (Production Example 3) | | | | | | | | | 12 | 8 | | | | | | |
| 8 | C | Methyl hydrogen polysiloxane-surface treated fine particle titanium dioxide powder (Production Example 4) | | | | | | | | | 8 | | | | | | | |
| 9 | | Glycerin | 2 | 2 | 2 | 2 | 2 | 2 | 2 | 2 | 2 | 2 | 2 | 2 | 2 | 2 | 2 | 2 |
| 10 | | 1,3-Butylene glycol | 1 | 1 | 1 | 1 | 1 | 1 | 1 | 1 | 1 | 1 | 1 | 1 | 1 | 1 | 1 | 1 |
| 11 | | Ethanol | 10 | 10 | 10 | 10 | 10 | 10 | 10 | 10 | 10 | 10 | 10 | 10 | 10 | 10 | 10 | 10 |

| | | | | | | | | | | | | | | | | | | |
|---|---|---|---|---|---|---|---|---|---|---|---|---|---|---|---|---|---|---|
| 12 | D | (Sodium acrylate/sodium acryloyldimethyl taurate) copolymer *3 | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 |
| 13 | D | Xanthan gum | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 |
| 14 | | Disodium edetate | 0.02 | 0.02 | 0.02 | 0.02 | 0.02 | 0.02 | 0.02 | 0.02 | 0.02 | 0.02 | 0.02 | 0.02 | 0.02 | 0.02 | 0.02 | 0.02 |
| 15 | | Pure water | Balance | Balance | Balance | Balance | Balance | Balance | Balance | Balance | Balance | Balance | Balance | Balance | Balance | Balance | Balance | Balance |
| | | | | | | | | | | | | | | | | | | |
| | | A/B | 0.076 | 0.076 | 0.076 | 0.076 | 0.076 | 0.379 | 0.161 | 0.044 | 0.076 | 0.076 | 0.008 | 0.038 | 0.606 | 0.038 | 1.039 | |
| | | A/C | 0.050 | 0.067 | 0.167 | 0.200 | 0.250 | 0.500 | 0.100 | 0.100 | 0.050 | 0.050 | 0.005 | 0.025 | 0.800 | 0.500 | 0.727 | |
| | | B/C | 0.660 | 0.880 | 2.200 | 2.640 | 3.300 | 1.320 | 0.620 | 2.270 | 0.660 | 0.660 | 0.660 | 0.660 | 1.320 | 13.200 | 0.700 | |
| Evaluation | | Uniform application evaluation | 3 | 3.5 | 3.2 | 3.3 | 3.5 | 3.5 | 5.8 | 3.6 | 3.1 | 3.0 | 5.3 | 4.2 | 4.8 | 3.2 | 5.1 | |
| | | UV protective effect | 4 | 3 | 4 | 4 | 1 | 3 | 1 | 3 | 4 | 4 | 4 | 4 | 3 | 1 | 2 | |
| | | Spreadability and staying property | 4.1 | 4.3 | 4.6 | 4.8 | 4.8 | 4.1 | 3.8 | 4.5 | 4 | 4.1 | 4.1 | 4.2 | 4 | 4.4 | 3.8 | |
| | | Feeling of moisture | 4.2 | 4.1 | 4.3 | 4.2 | 4.3 | 4.6 | 4.2 | 4.6 | 4.2 | 4.3 | 4.1 | 4.2 | 4.6 | È | 4.5 | |
| | | Feeling of stickiness | 4.6 | 4.6 | 4.4 | 4.5 | 4.4 | 4 | 4.5 | 4.1 | 4.5 | 4.4 | 4.2 | 4.3 | 4 | 4.3 | 4.2 | |
| | | Feeling of friction | 4 | 4.1 | 4.3 | 4.8 | 4.8 | 4.1 | 4 | 4.5 | 4 | 4 | 3.8 | 4 | 4 | 4.8 | 3.8 | |

(Production method)

a: The components (1) to (8) are dissolved by heating at 70°C and uniformly mixed.
b: The components (9) to (15) are uniformly mixed at room temperature (25°C ± 3°C).
c: While the product obtained in the step b is stirred, the product obtained in the step a is gradually added thereto. The mixture is uniformly mixed.
d: The mixture obtained in the step c is cooled to room temperature.

The samples of Examples 2 to 16 obtained excellent scores in all of the evaluations on spreadability and staying property, a feeling of moisture, the absence of a feeling of stickiness, and the absence of a feeling of friction. Even the samples of Examples having lower UV protective ability compared with the sample of Reference Example 1 in the test on UV protective ability in Table 2 can be preferably used as sunscreens for daily use which does not require much UV protective ability.

Hereinafter, Formulation Example of the emulsion composition of the present invention will be given. The formulation is expected to spread and stay well, offer reduced feelings of friction and stickiness, be excellent in stability and UV protective ability, and also exhibit an excellent moisturizing effect.

Formulation Example 1 (oil-in-water sunscreen) Components

| [Component (A)] | Content (% by mass) |
|---|---|
| Polyoxyethylene (2) behenyl ether | 1 |
| [Component (B)] | |
| 2-Ethylhexyl p-methoxycinnamate | 8.0 |
| Octyldodecyl myristate | 1.0 |
| Dimethylpolysiloxane (6 $mm^2/s$) | 4.5 |
| [Component (C)] | |
| Octyltriethoxysilane-surface treated flaky powder of zinc oxide (Production Example 1) | 4.0 |
| Methyl hydrogen polysiloxane-surface treated fine particle zinc oxide powder (Production Example 2) | 5.0 |
| [Component (D)] | |
| (Sodium acrylate/sodium acryloyldimethyl taurate) copolymer | 2.5 |
| Xanthan gum | 0.1 |
| Hyaluronic acid | 0.1 |
| [Component F] | |
| Glycerin | 0.5 |
| 1,3-Butylene glycol | 1.5 |
| [Component E] | |
| Ethanol | 10 |
| [Other components] | |
| Disodium edetate | 0.02 |
| Phenoxyethanol | 0.1 |
| Alga extract (manufactured by Maruzen Pharmaceuticals Co., Ltd.; Alga Extract M) | 0.1 |
| Hydrolyzed collagen (manufactured by Seiwa Kasei Co., Ltd.; PROMOIS WU-32R) | 0.1 |
| Royal jelly extract (manufactured by Ichimaru Pharcos Co., Ltd.; Royal Jelly Extract) | 0.1 |
| Job's tears (*Coix lacryma-jobi* var. *ma-yuen*) seed extract (manufactured by Maruzen Pharmaceuticals Co., Ltd.; Coix Seed Extract BG-S) | 0.1 |
| Firethorn (*Pyracantha fortuneana*) extract (manufactured by Maruzen Pharmaceuticals Co., Ltd.; Firethorn) | 0.1 |

(continued)

| [Component (A)] | Content (% by mass) |
|---|---|
| Watercress (*Nasturtium officinale*) extract (manufactured by Silab; WaterCress-KB) | 0.1 |
| Pure water | Balance |

## Claims

**1.** An oil-in-water emulsion composition comprising the following components (A) to (C):

(A) a polyoxyethylene alkyl or alkenyl ether which has an alkyl or alkenyl group having 20 or more and 24 or less carbon atoms and has an average molar number of ethylene oxide added of 1.5 or more and 4 or less,
(B) a liquid oil, and
(C) a hydrophobized fine particle metal oxide powder,

wherein the content mass ratio of the component (A) to the component (C), (A/C), is 0.01 or more and 0.7 or less..

**2.** The oil-in-water emulsion composition according to claim 1, wherein the content of the component (C) with respect to the total amount of the composition is 0.1% by mass or more and 30% by mass or less.

**3.** The oil-in-water emulsion composition according to claim 1 or 2, wherein the component (C) is a hydrophobization product of one or two or more metal oxide powders selected from the group consisting of zinc oxide, titanium dioxide, and cerium oxide.

**4.** The oil-in-water emulsion composition according to any one of claims 1 to 3, wherein a hydrophobization treatment of the fine particle metal oxide powder is at least one surface treatment selected from a surface treatment using silicone or a silicone resin and a surface treatment using methyl hydrogen polysiloxane.

**5.** The oil-in-water emulsion composition according to any one of claims 1 to 4, wherein the component (B) comprises a liquid organic ultraviolet absorber and an oil agent for a cosmetic composition.

**6.** The oil-in-water emulsion composition according to claim 5, wherein the liquid organic ultraviolet absorber is at least one selected from the group consisting of 2-ethylhexyl p-methoxycinnamate, 2-ethoxyethyl p-methoxycinnamate, an isopropyl p-methoxycinnamate-diisopropyl cinnamic acid ester mixture, methyl bis(trimethylsiloxy)silylisopentyl trimethoxycinnamate, amyl p-dimethylaminobenzoate, 2-ethylhexyl p-dimethylaminobenzoate, ethylene glycol salicylate, 2-ethylhexyl salicylate, benzyl salicylate, homomenthyl salicylate, octocrylene, and dimethicodiethyl benzalmalonate.

**7.** The oil-in-water emulsion composition according to any one of claims 1 to 6, wherein the content of the component (B) with respect to the total amount of the composition is 0.5% by mass or more and 30% by mass or less.

**8.** The oil-in-water emulsion composition according to any one of claims 1 to 7, further comprising (D) a watersoluble polymer.

**9.** The oil-in-water emulsion composition according to claim 8, wherein the component (D) is one or two or more selected from the group consisting of carboxyvinyl polymer, acrylic acid-alkyl methacrylate copolymer, xanthan gum, hydroxypropylmethylcellulose, polyacrylamide, (sodium acrylate/sodium acryloyldimethyl taurate) copolymer, and hyaluronic acid or an alkali metal salt thereof.

**10.** The oil-in-water emulsion composition according to any one of claims 1 to 9, wherein the content mass ratio of the component (A) to the component (B), (A/B), is 0.02 or more and 1 or less.

**11.** The oil-in-water emulsion composition according to any one of claims 1 to 10, wherein the oil-in-water emulsion composition has an average emulsified particle size of from 1 to 30 μm.

**12.** Use of an oil-in-water emulsion composition according to any one of claims 1 to 11 as a sunscreen skin preparation for external use.

**13.** A method for producing an oil-in-water emulsion composition, comprising dissolving an oil-phase component containing the following components (A) to (C) by heating at a temperature exceeding 45°C and emulsifying the oil-phase component with an aqueous-phase component having a temperature of 15 to 40°C:

(A) a polyoxyethylene alkyl or alkenyl ether which has an alkyl or alkenyl group having 20 or more and 24 or less carbon atoms and has an average molar number of ethylene oxide added of 1.5 or more and 4 or less,
(B) a liquid oil, and
(C) a hydrophobized fine particle metal oxide powder.

**14.** The method for producing an oil-in-water emulsion composition according to claim 13, wherein the emulsification is performed by the gradual addition of the oil-phase component into the aqueous-phase component.

**Patentansprüche**

**1.** Öl-in-Wasser-Emulsionszusammensetzung, umfassend die folgenden Komponenten (A) bis (C):

- (A) einen Polyoxyethylenalkyl oder -Alkenylether, der eine Alkyl- oder Alkenylgruppe mit 20 oder mehr und 24 oder weniger Kohlenstoffatomen aufweist und eine durchschnittliche Molzahl von hinzugefügtem Ethylenoxid von 1,5 oder mehr und 4 oder weniger aufweist,
- (B) ein flüssiges Öl, und
- (C) ein hydrophobiertes feinteiliges Metalloxidpulver,

wobei das Gehaltsmassenverhältnis der Komponente (A) zu der Komponente (C), (A/C), 0,01 oder mehr und 0,7 oder weniger beträgt.

**2.** Öl-in-Wasser-Emulsionszusammensetzung nach Anspruch 1, wobei der Gehalt der Komponente (C) in Bezug auf die Gesamtmenge der Zusammensetzung 0,1 Massen-% oder mehr und 30 Massen-% oder weniger beträgt.

**3.** Öl-in-Wasser-Emulsionszusammensetzung nach Anspruch 1 oder 2, wobei die Komponente (C) ein Hydrophobierungsprodukt aus einem oder zwei oder mehreren Metalloxidpulvern ist, ausgewählt aus der Gruppe bestehend aus Zinkoxid, Titandioxid und Ceroxid.

**4.** Öl-in-Wasser-Emulsionszusammensetzung nach einem der Ansprüche 1 bis 3, wobei eine Hydrophobierungsbehandlung des feinteiligen Metalloxidpulvers mindestens eine Oberflächenbehandlung ist, ausgewählt aus einer Oberflächenbehandlung unter Verwendung von Silikon oder einem Silikonharz und einer Oberflächenbehandlung unter Verwendung von Methylhydrogenpolysiloxan.

**5.** Öl-in-Wasser-Emulsionszusammensetzung nach einem der Ansprüche 1 bis 4, wobei die Komponente (B) einen flüssigen organischen Ultraviolettabsorber und ein Ölmittel für eine kosmetische Zusammensetzung umfasst.

**6.** Öl-in-Wasser-Emulsionszusammensetzung nach Anspruch 5, wobei der flüssige organische Ultraviolettabsorber mindestens eines ist, ausgewählt aus der Gruppe bestehend aus 2-Ethylhexyl-p-methoxycinnamat, 2-Ethoxyethyl-p-methoxycinnamat, einem Isopropyl-p-methoxycinnamat-düsopropyl Zimtsäureester-Gemisch, Methyl-bis (trimethylsiloxy) silylisopentyltrimethoxycinnamat, Amyl-p-dimethylaminobenzoat, 2-Ethylhexyl-p-dimethylaminobenzoat, Ethylenglykolsalicylat, 2-Ethylhexylsalicylat, Benzylsalicylat, Homomenthylsalicylat, Octocrylen und Dimethyldiethylbenzalmalonat.

**7.** Öl-in-Wasser-Emulsionszusammensetzung nach einem der Ansprüche 1 bis 6, wobei der Gehalt der Komponente (B) in Bezug auf die Gesamtmenge der Zusammensetzung 0,5 Massen-% oder mehr und 30 Massen-% oder weniger beträgt.

**8.** Öl-in-Wasser-Emulsionszusammensetzung nach einem der Ansprüche 1 bis 7, weiter umfassend (D) ein wasserlösliches Polymer.

**9.** Öl-in-Wasser-Emulsionszusammensetzung nach Anspruch 8, wobei die Komponente (D) eines oder zwei oder mehreres ist, ausgewählt aus der Gruppe bestehend aus Carboxyvinylpolymer, Acrylsäure-Alkylmethacrylat-Copolymer, Xanthangummi, Hydroxypropylmethylcellulose, Polyacrylamid (NatriumAcrylat/Natriumacryloyldimethyltaurat)-Copolymer und Hyaluronsäure oder ein Alkalimetallsalz davon.

**10.** Öl-in-Wasser-Emulsionszusammensetzung nach einem der Ansprüche 1 bis 9, wobei das Gehaltsmassenverhältnis der Komponente (A) zu der Komponente (B), (A/B), 0,02 oder mehr und 1 oder weniger beträgt.

**11.** Öl-in-Wasser-Emulsionszusammensetzung nach einem der Ansprüche 1 bis 10, wobei die Öl-in-Wasser-Emulsionszusammensetzung eine mittlere emulgierte Teilchengröße von 1 bis 30 $\mu$m aufweist.

**12.** Verwendung von einer Öl-in-Wasser-Emulsionszusammensetzung nach einem der Ansprüche 1 bis 11 als ein Sonnenschutz-Hautpräparat zur äußeren Verwendung.

**13.** Verfahren zum Herstellen einer Öl-in-Wasser-Emulsionszusammensetzung, umfassend das Auflösen einer Öl-Phasen-Komponente, die die folgenden Komponenten (A) bis (C) enthält, durch Erwärmen bei einer Temperatur von mehr als 45 °C und Emulgieren der Öl-Phasen-Komponente mit einer Wasser-Phasen-Komponente, die eine Temperatur von 15 bis 40 °C aufweist:

- (A) einen Polyoxyethylenalkyl oder -Alkenylether, der eine Alkyl- oder Alkenylgruppe mit 20 oder mehr und 24 oder weniger Kohlenstoffatomen aufweist und eine durchschnittliche Molzahl von hinzugefügtem Ethylenoxid von 1,5 oder mehr und 4 oder weniger aufweist,
- (B) ein flüssiges Öl, und
- (C) ein hydrophobiertes feinteiliges Metalloxidpulver.

**14.** Verfahren zum Herstellen einer Öl-in-Wasser-Emulsionszusammensetzung nach Anspruch 13, wobei die Emulgierung durch schrittweises Hinzufügen der Öl-Phasen-Komponente zu der Wasser-Phasen-Komponente durchgeführt wird.

## Revendications

**1.** Composition d'émulsion huile-dans-eau comprenant les composants (A) à (C) suivants :

(A) un éther d'alkyle ou d'alcényle de polyoxyéthylène qui a un groupe alkyle ou alcényle qui possède 20 ou plus et 24 ou moins atomes de carbone et possède un nombre molaire moyen d'oxyde d'éthylène ajouté de 1,5 ou plus et de 4 ou moins,
(B) une huile liquide, et
(C) une poudre d'oxyde métallique à particules fines hydrophobisées, dans laquelle le ratio massique de teneurs du composant (A) par rapport au composant (C), (A/C), est de 0,01 ou plus et de 0,7 ou moins.

**2.** Composition d'émulsion huile-dans-eau selon la revendication 1, dans laquelle la teneur en composant (C) par rapport à la quantité totale de la composition est de 0,1 % en masse ou plus et de 30 % en masse ou moins.

**3.** Composition d'émulsion huile-dans-eau selon la revendication 1 ou 2, dans laquelle le composant (C) est un produit d'hydrophobisation d'une ou deux poudres d'oxyde métallique, ou plus, choisies dans le groupe constitué par l'oxyde de zinc, le dioxyde de titane et l'oxyde de cérium.

**4.** Composition d'émulsion huile-dans-eau selon l'une quelconque des revendications 1 à 3, dans laquelle un traitement d'hydrophobisation de la poudre d'oxyde métallique à particules fines est au moins un traitement de surface choisi parmi un traitement de surface utilisant un silicone ou une résine de silicone et un traitement de surface utilisant du méthyl-hydrogène-polysitoxane.

**5.** Composition d'émulsion huile-dans-eau selon l'une quelconque des revendications 1 à 4, dans laquelle le composant (B) comprend un absorbeur d'ultraviolets organique liquide et un agent huileux pour une composition cosmétique.

**6.** Composition d'émulsion huile-dans-eau selon la revendication 5, dans laquelle l'absorbeur d'ultraviolets organique liquide est au moins un choisi dans le groupe constitué par le p-méthoxycinnamate de 2-éthylhexyle, le p-méthoxycinnamate de 2-éthoxyéthyle, un mélange de p-méthoxycinnamate d'isopropyle et d'ester d'acide cinnamique de diisopropyl, le triméthoxycinnamate de méthyl-bis(triméthylsiloxy)silylisopentyle, le p-diméthylaminobenzoate d'amyle, le p-diméthylaminobenzoate de 2-éthylhexyle, le salicylate d'éthylène glycol, le salicylate de 2-éthylhexyle,

le salicylate de benzyle, le salicylate d'homomenthyle, l'octocrylène et le benzalmalonate de diméthicodiéthyle.

7. Composition d'émulsion huile-dans-eau selon l'une quelconque des revendications 1 à 6, dans laquelle la teneur en composant (B) par rapport à la quantité totale de la composition est de 0,5 % en masse ou plus et de 30 % en masse ou moins.

8. Composition d'émulsion huile-dans-eau selon l'une quelconque des revendications 1 à 7, comprenant en outre (D) un polymère soluble dans l'eau.

9. Composition d'émulsion huile-dans-eau selon la revendication 8, dans laquelle le composant (D) est un ou deux composés, ou plus, choisis dans le groupe consistant en un polymère de carboxyvinyle, un copolymère d'acide acrylique-méthacrylate d'alkyle, une gomme de xanthane, une hydroxypropylméthylcellulose, un polyacrylamide, un copolymère (acrylate de sodium/taurate d'acryloyldiméthyle de sodium), et l'acide hyaluronique ou un sel de métal alcalin de ceux-ci.

10. Composition d'émulsion huile-dans-eau selon l'une quelconque des revendications 1 à 9, dans laquelle le ratio massique de teneurs du composant (A) par rapport au composant (B), (A/B), est de 0,02 ou plus et de 1 ou moins.

11. Composition d'émulsion huile-dans-eau selon l'une quelconque des revendications 1 à 10, dans laquelle la composition d'émulsion huile-dans-eau a une granulométrie émulsifiée moyenne de 1 à 30 μm.

12. Utilisation d'une composition d'émulsion huile-dans-eau selon l'une quelconque des revendications 1 à 11 en tant que préparation solaire pour la peau pour une utilisation externe.

13. Procédé de fabrication d'une composition d'émulsion huile-dans-eau, comprenant la dissolution d'un composant en phase huileuse contenant les composants (A) à (C) suivants en chauffant à une température supérieure à 45 °C et en émulsifiant le composant en phase huileuse avec un composant en phase aqueuse ayant une température de 15 à 40 °C :

(A) un éther d'alkyle ou alcényle de polyoxyéthylène qui a un groupe alkyle ou alcényle qui possède 20 ou plus et 24 ou moins atomes de carbone et qui possède un nombre molaire moyen d'oxyde d'éthylène ajouté de 1,5 ou plus et de 4 ou moins,
(B) une huile liquide, et
(C) une poudre d'oxyde métallique à particules fines hydrophobisées.

14. Procédé de fabrication d'une composition d'émulsion huile-dans-eau selon la revendication 13, dans lequel l'émulsification est effectuée par l'addition graduelle du composant en phase huileuse dans le composant en phase aqueuse.

Figure 1

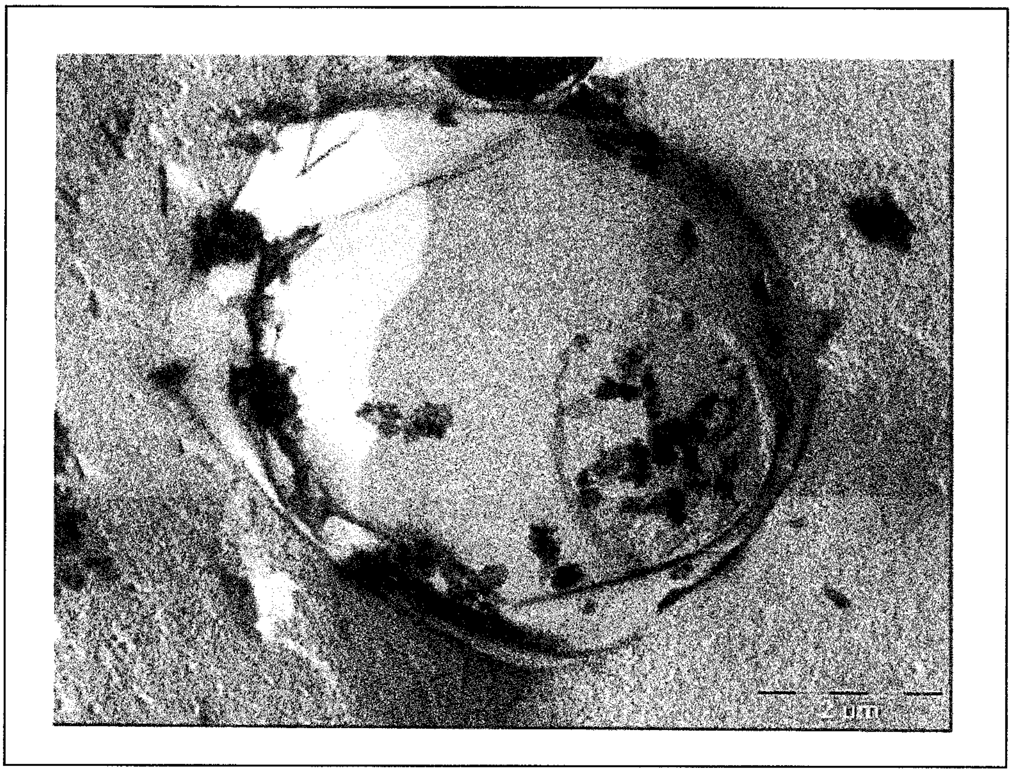

Figure 2

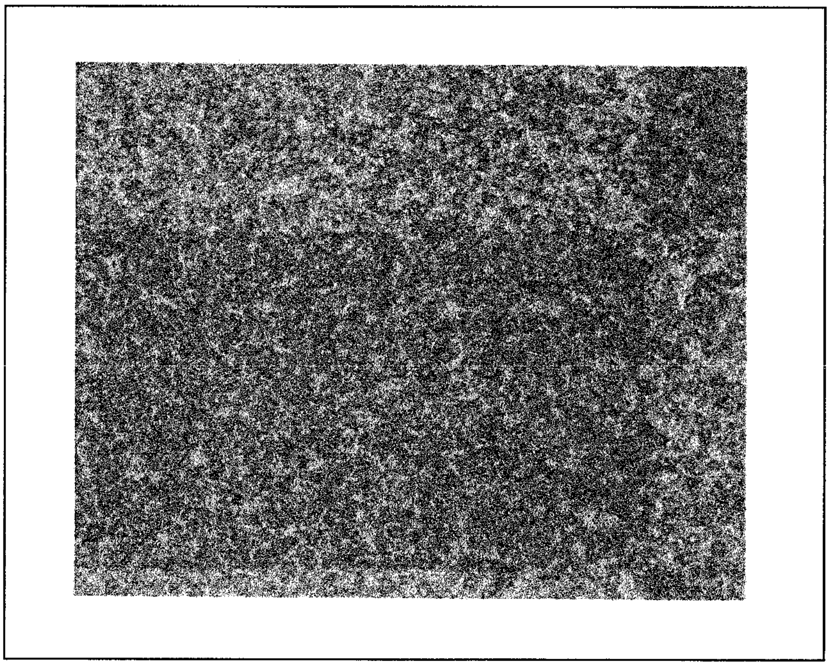

Figure 3

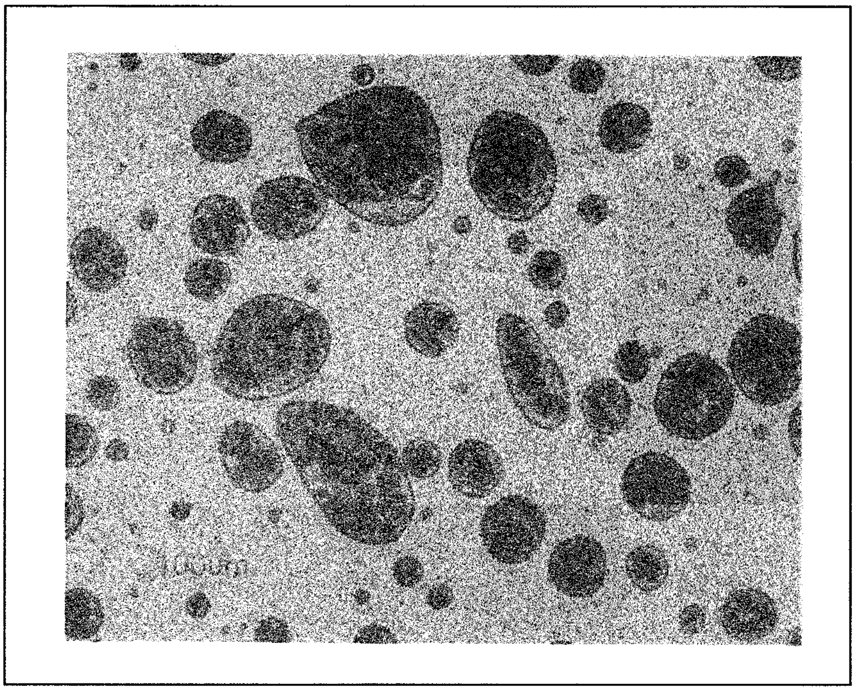

Figure 4

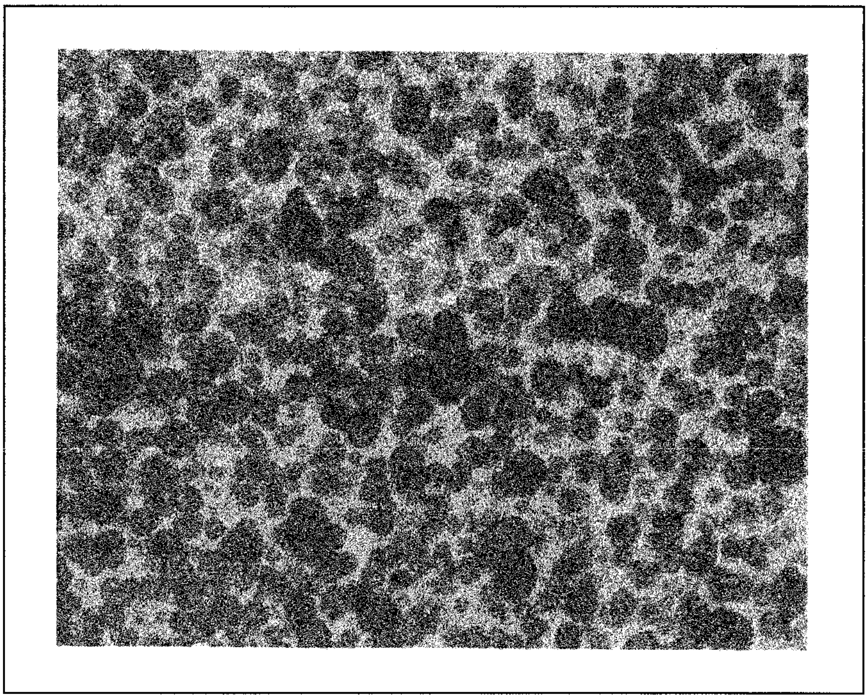

Figure 5

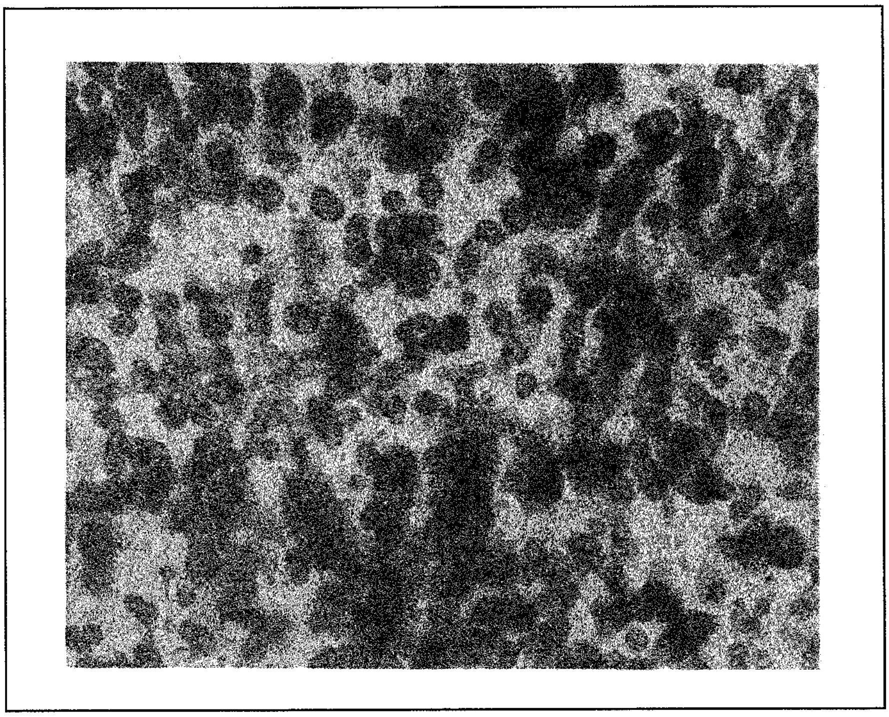

Figure 6

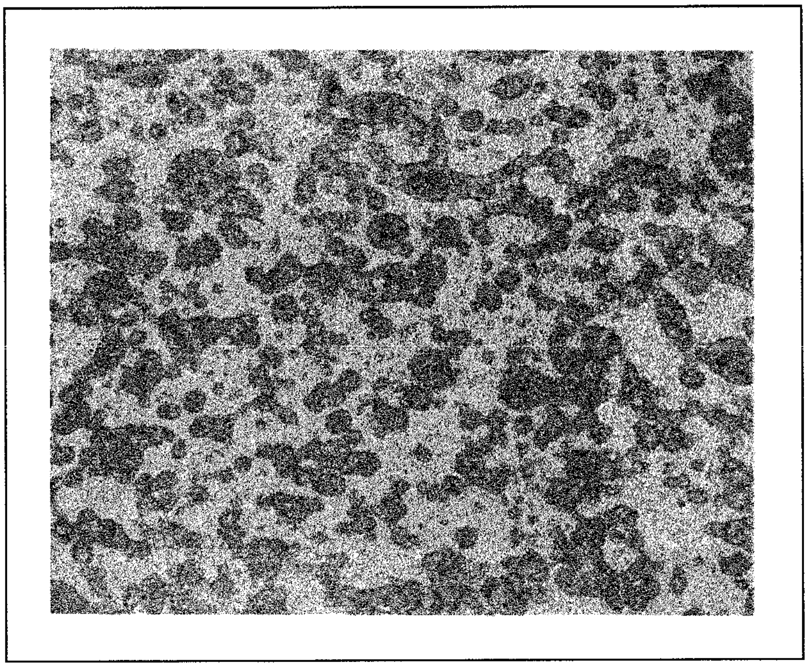

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2006008796 A
- JP 2005272389 A
- WO 2011136121 A
- WO 2011155404 A
- JP 3187440 B
- JP 2007326902 A